# EUROPEAN PATENT SPECIFICATION

(11) **EP 4 512 436 B1**
(45) Date of publication and mention of the grant of the patent: **04.03.2026**
(21) Application number: 23202385.3
(22) Date of filing: 09.10.2023
(51) Int. Cl.: A61L 24/00, A61L 24/02, A61L 24/10, A61L 27/24, A61L 27/46, A61L 27/54, A61L 27/58, C08L 89/06, A61K 31/7012

(54) **BONE GRAFT COMPOSITION BASED ON GLUCURONIC ACID-1-PHOSPHATE**
KNOCHENTRANSPLANTATZUSAMMENSETZUNG AUF BASIS VON GLUCURONSÄURE-1-PHOSPHAT
COMPOSITION DE GREFFE OSSEUSE À BASE D'ACIDE GLUCURONIQUE-1-PHOSPHATE

(30) Priority: 21.08.2023 EP 23192445
(43) Date of publication of application: 26.02.2025
(73) Proprietor: DENTSPLY SIRONA Inc., York, PA 17401 (US); Sirona Dental Systems GmbH, 64625 Bensheim (DE)
(72) Inventor: CARTIER, Régis, 64625 Bensheim (DE)
(74) Representative: Crow, Martin

(56) References cited:
- US-A1- 2012 082 705
- ALINA KIRILLOVA ET AL: "Bioinspired Mineral-Organic Bioresorbable Bone Adhesive", ADVANCED HEALTHCARE MATERIALS, WILEY - V C H VERLAG GMBH & CO. KGAA, DE, vol. 7, no. 17, 25 June 2018 (2018-06-25), pages n/a, XP072463163, ISSN: 2192-2640, DOI: 10.1002/ADHM.201800467
- NORTON MICHAEL R ET AL: "Bone glue - The final frontier for fracture repair and implantable device stabilization", INTERNATIONAL JOURNAL OF ADHESION AND ADHESIVES, ELSEVIER, AMSTERDAM, NL, vol. 102, 15 May 2020 (2020-05-15), XP086245211, ISSN: 0143-7496, [retrieved on 20200515], DOI: 10.1016/J.IJADHADH.2020.102647

## Description

### BACKGROUND OF THE INVENTION

The prior art compositions comprising bone graft materials have low biocompatibility and very often demonstrate low or moderate bone connection.

In the field of dental applications, bone growth products and adhesive materials each have their own unique advantages and disadvantages. Bone growth products are typically well-tolerated by the body, making them highly biocompatible. They provide a scaffold for new bone growth, a crucial factor for successful bone regeneration. Additionally, these materials maintain their volume over time, which is important for the success of the procedure. They have been widely used and studied, with many clinical trials demonstrating their effectiveness.

However, there are also some drawbacks to consider. Some of these products are derived from animals, which may raise concerns about disease transmission. Although the risk is extremely low due to rigorous processing and sterilization, it is still a factor to consider. These products can also be more expensive than some other grafting materials. Additionally, they may resorb slowly, which can be a disadvantage in some cases where faster bone regeneration is desired.

On the other hand, adhesive materials used in dental applications offer strong adhesion, providing a robust bond that can withstand the forces in the oral environment. Like bone growth products, they are well-tolerated by the body, making them biocompatible. They are versatile and can be used with a variety of materials, including metal, ceramic, and natural bone. Another advantage is their rapid setting, which can be beneficial in surgical settings.

However, adhesive materials used in dental applications, while beneficial, do come with their own set of potential drawbacks. While many of these materials are designed to be biocompatible, pH issues occur, leading to inflammation reactions and cytotoxic effects.

Another issue is the degradation of the adhesive over time. If the product degrades too quickly, it will lead to a loss of bond strength and the need for replacement or repair. It is why the degradation of adhesive material is designed to be slow, resulting in the replacement of the natural bone taking a lot of time, which can, however, increase the risk of complications and overloaded implant.

The application of these adhesives can also represent a challenge to apply them precisely, especially in the limited space of the oral cavity. This could potentially lead to issues with the fit and positioning of the dental implant.

It is an object of the present invention to overcome the above shortcomings.

US2012082705A1 discloses compositions and methods of their use to adhere a variety of materials together. The compositions include at least tetra calcium phosphate, an effective amount of a compound that is structurally similar to phosphoserine, and can be mixed with an aqueous solution.

### Terms and Abbreviations used in the present invention:

### Glucuronic acid-1-phosphate (GA1PH) is a compound of the following formula:

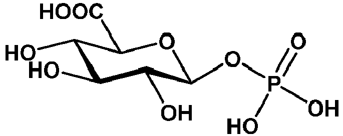

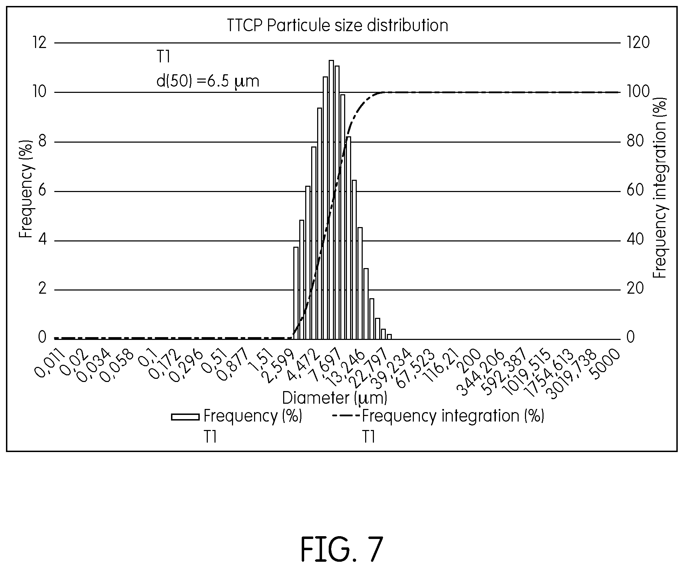

**ISQ=** Implant Stability Quotient; its value is on a scale from 1 to 100; it measures implant stability. The ISQ scale has a non-linear correlation with micro mobility.
**TTCP** = tetracalcium phosphate, **Ca4** (PO4 )2 O
**Xcoll** = crosslinked collagen
**Ca/P** = ratio of Calcium/Phosphorus atoms in tetracalcium phosphate, i.e. a ratio of a number of Calcium and Phosphorus atoms in tetracalcium phosphate
**L/P** = weight ratio Liquid/Powder, e.g. weight ratio Water/Powder
**BGM** = bone graft material
**Adhesive/mineral ratio** = weight ratio between glucuronic acid-1-phosphate and tetracalcium phosphate
**Setting time** = defined with a Gillmore needle, the setting time represents the time elapsed from mixing to the time at which no indentation can be detected on the mixture surface. The initial setting time and final setting time were determined corresponding to the ASTM C266-03 (2008) and ISO9917.
**d(50)** = A median diameter of a particle of a powder. 50% of the particles are below the size d and 50 % of the particles are above the size d (See Fig. 7).
**Drug** in the sense of the present invention is a medical drug.
**Bio-Oss^{®}** = Spongious bone substitute from bovine derivatives of a company called Geistlich

### Description of the Figures

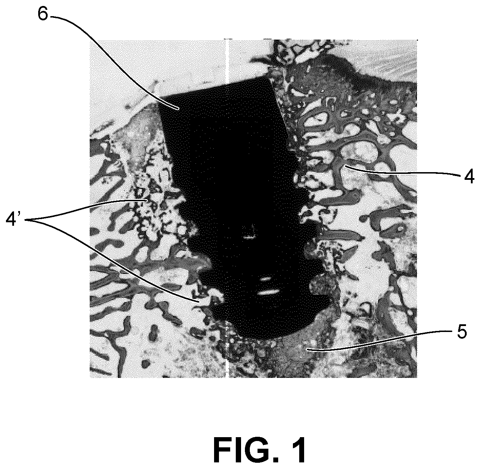

**Fig. 1** depicts histological results: the excellent bone connection with the composition of the present invention (a bone graft material) (5) after 12 weeks of bone-to-implant contact. The bone graft material was placed with an implant (6) in an animal model for 12 weeks. The implant is illustrated in black, there is a bone (4); a new bone (4') is showing a good structural integration. The composition of the present invention, a bone graft material (5) is integrated in the new bone (4').

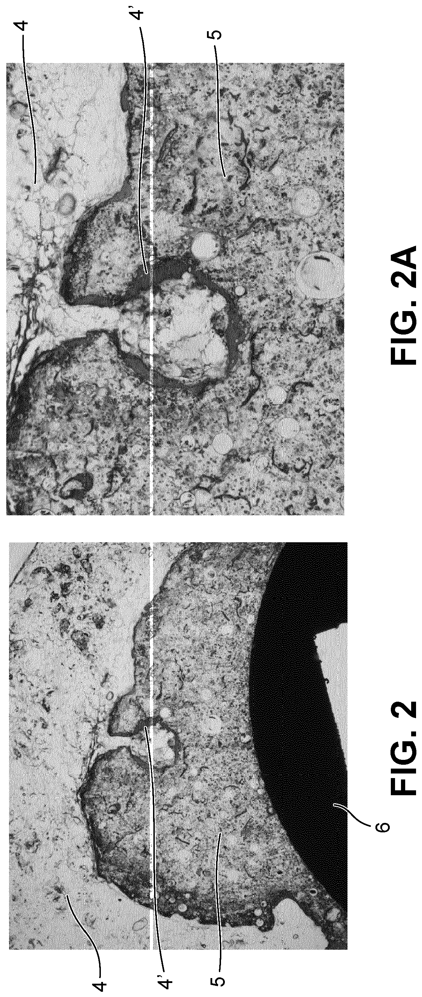

**Figs. 2 and 2a** depict histological results: the excellent bone connection with the composition of the present invention (a bone graft material) (5) after 12 weeks of bone-to-implant contact. The bone graft material was placed with an implant (6) in an animal model for 12 weeks. The implant is illustrated in black, there is a bone (4); a new bone (4') is showing a good structural integration. The composition of the present invention, a bone graft material (5) is integrated in the new bone (4').

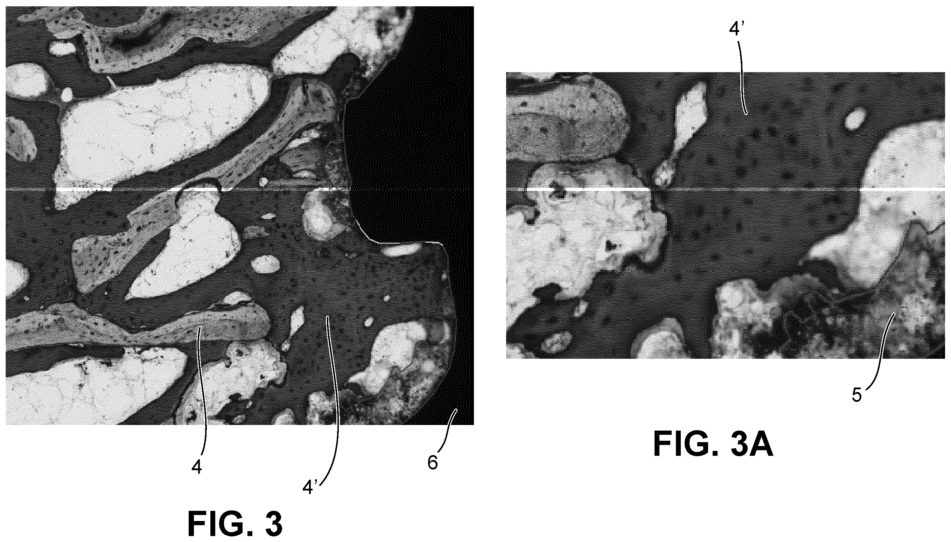

**Figs. 3 and 3a** depict histological results: the excellent bone connection with the composition of the present invention (a bone graft material) (5) after 12 weeks of bone-to-implant contact. The bone graft material (5) was placed with an implant (6) in an animal model for 12 weeks. The implant is illustrated in black (6), there is a new bone (4'). The composition of the present invention, a bone graft material is integrated in the new bone (4'), (4) illustrates an old bone.

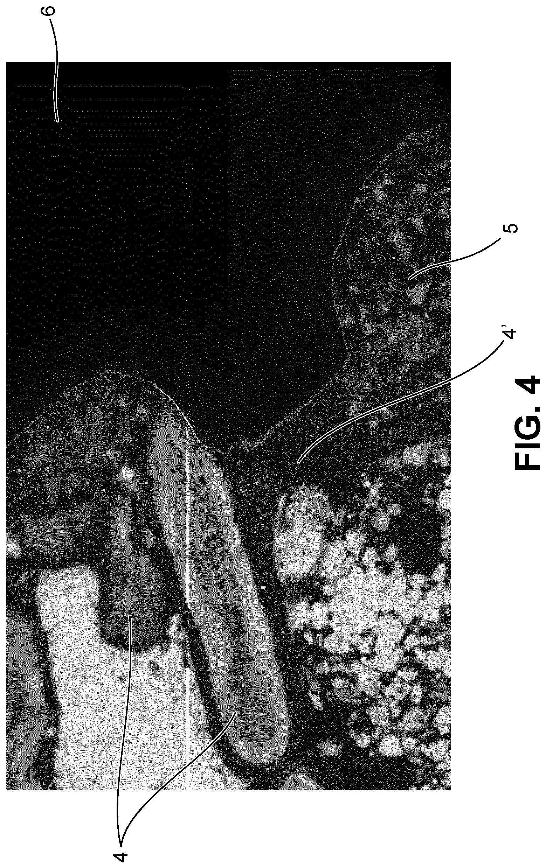

**Fig. 4** depicts histological results: the excellent bone connection with the composition of the present invention (a bone graft material) (5) after 12 weeks of bone-to-implant contact. The bone graft material was placed with an implant (6) in an animal model for 12 weeks. The implant is illustrated in black, there is a new bone (4'). The composition of the present invention, a bone graft material (5) is integrated in the new bone (4'). There is also an old bone (4).

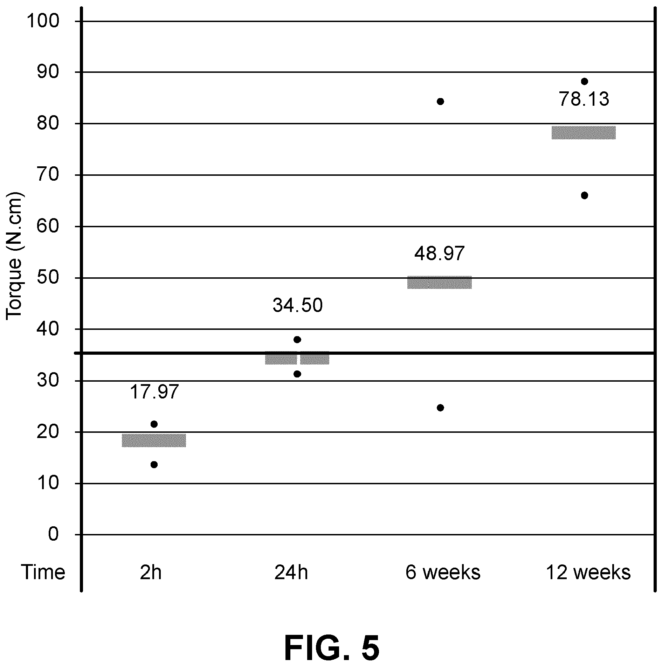

**Fig. 5** depicts results of the Reverse Torque (N.cm) test of the implant stabilized with the composition of the present invention i.e. the necessary torque to remove the implant. The Reverse Torque was measured after 2 h, 24 h, 6 weeks and 12 weeks, calculated from the application of the composition of the present invention. Reverse Torque of 35 N.cm is a target value for immediate provisional loading. The dots represent the extremities of the standard deviation, while the bars represent the mean of the measurements. The Reverse Torque was measured using the torque gauges Serie TT03 from Mark-10. Using the abutment part to fix the device, the gauge was manually rotated to measure the maximum torque needed to remove the implant.

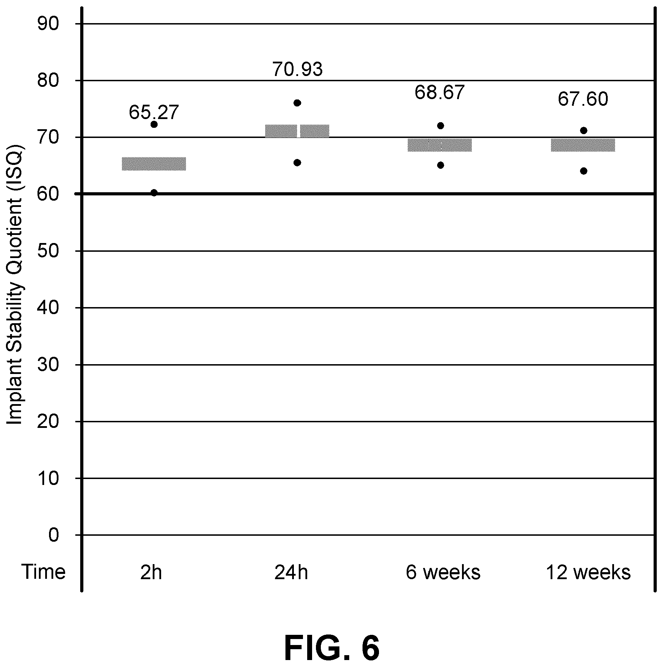

**Fig. 6** depicts the results of the ISQ test of the implant stabilized with the composition of the present invention after 2 h, 24 h, 6 weeks and 12 weeks, calculated from the application of the composition of the present invention. ISQ of 60 is a target value for immediate provisional loading. The dots represent the extremities of the standard deviation, while the bars represent the mean of the measurements.
**Fig. 7** depicts tetracalcium phosphate particle size distribution. For the tetracalcium phosphate, the d(50) is preferably ≤ 10µm, which gives the composition of the present invention good mechanical properties and appropriate setting time. d(50) is a median diameter of a particle of a powder. 50% of the particles are below the size d and 50 % of the particles are above the size d. The particle size was measured by laser granulometry.

In the above description of Figures 1-6, the composition of the present invention is in particular a composition comprising: glucuronic acid-1-phosphate, tetracalcium phosphate, a cross-linked collagen and water and more preferably as defined in Example 1.

### DETAILED DESCRIPTION OF THE INVENTION

It has been found that compositions based on glucuronic acid-1-phosphate give surprisingly good results in respect of biocompatibility and bone connection.

It is an object of the present invention to present use of glucuronic acid-1-phosphate in the dental and medical practice. The scope of this invention is defined by the claims, and any combination of features not falling within the scope of the claims is only disclose as illustrative purpose.

It is an object of the present invention to provide a composition comprising glucuronic acid-1-phosphate, TTCP, a cross-linked collagen and water. Water is used to make the composition injectable.

In the preferred embodiment of the present invention, the cross-linked collagen is a sugar cross-linked collagen.

In the preferred embodiment of the present invention, the cross-linked collagen is grinded and sieved to the particle size of 80 µm. The said particle size has a positive effect on the properties of the composition of the present invention, including absorption and biocompatibility. The particle size was measured by laser granulometry.

In the preferred embodiment of the present invention, the composition comprises 40-70 weight % of TTCP, 15-40 weight % of glucuronic acid-1-phosphate, 10-25 weight % of water and 0.1-5.0 weight % of cross-linked collagen, based on the total weight of the composition.

In a more preferred embodiment of the present invention, the composition comprises 50-60 weight % of TTCP, 25-37 weight % of glucuronic acid-1-phosphate, 12-20 weight % of water and 1-2.5 weight % of cross-linked collagen, based on the total weight of the composition.

In a yet more preferred embodiment of the present invention, the composition comprises 51.4 weight % of TTCP, 32.5 weight % of glucuronic acid-1-phosphate, 15.3 weight % of water and 0.88 weight % of cross-linked collagen, based on the total weight of the composition.

In the preferred embodiment of the present invention, the weight ratio the glucuronic acid-1-phosphate and TTCP, i.e. the adhesive/mineral ratio is 0.63, which gives good mechanical and adhesive properties to the composition. The above weight ratio also makes it possible to obtain a final setting time of less than or equal to 30 minutes. TTCP is a mineral part of the composition.

In the preferred embodiment of the present invention, the weight ratio liquid/powder, i.e. the weight ratio water/powder is 0.18. Reduction in the liquid/powder weight ratio when compared with the state of the art results in the reduction in the setting time. Powder in the sense of this invention includes all powder components of the composition, i.e. TTCP, glucuronic acid-1-phosphate and cross-linked collagen.

In the preferred embodiment of the present invention, the Ca/P ratio of the mineral part of the composition, i.e. tetracalcium phosphate, is 2, which is close to that of the natural bone. The Ca/P ratio is a ratio of the number of calcium atoms and the number of phosphorus atoms in tetracalcium phosphate. Tetracalcium phosphate is a mineral part of the composition.

For the TTCP, the d(50) is preferably ≤ 10 µm, which gives the composition good mechanical properties and appropriate setting time. The Fig. 7 depicts the particle size distribution of TTCP. In one of the embodiments of the present invention, the d(50) is 6.5 µm. The particle diameter was measured by laser granulometry.

The composition of the present invention can be used in producing a bone graft material and/or in producing a bone graft.

The composition of the present invention can be used in combination with another bone graft material in producing a bone graft.

The composition of the present invention can be used as an additive to a material used for tissue regeneration. In particular, it can be used as an adhesive in order to improve the mechanical stability of the used material. It can be also used to provide mechanical stability to another bone graft material. Furthermore, it can be used when a rapid dissolution or resorption of the material is needed in addition to stimulation of the formation of a new bone: to provide primary stability. The composition can be used in provisional restoration or a bone graft.

Compared with Bio-Oss^{®} , which represents the state of the art in terms of bone substitutes for regenerative dentistry, this invention makes it possible to obtain a composition that can be modellable and provides immediate primary stability during implant fixation. Indeed, Bio-Oss^{®} does not provide immediate primary stability and is composed of granules that can move and, when resorbed, generate bone outgrowths.

Possible dental applications of the composition of the present invention include stabilization of a dental implant during an immediate implant placement procedure when there is not sufficient bone to support the implant. This could be applied for a single root or a molar extraction. The composition could also be used in combination with other bone graft materials.

Another possible application of the composition of the present invention is dental ridge preservation in which the composition of the present invention entirely fills the ridge due to its fluidity and subsequently acts as a stable filler after solidification. Hence, the composition would not move and provide volume stability.

Another possible application of the composition of the present invention is the treatment of vertical and horizontal bone loss. In these cases, as the composition of the present invention self-hardens rapidly, it may or may not be covered by a bio-membrane.

Another possible application of the composition of the present invention is the treatment of larger bone defects in oral and maxillofacial surgery in which the composition of the present invention is used to cover and stabilize a material used as a main filler of the defect. In this case, the solidified composition of the present invention would hold the filler material in place by providing a wall- and/or upper-shell-like structure.

Another possible application of the composition of the present invention is the structural stabilization of a soft bio-membrane comprising a synthetic or a natural polymer, wherein the soft bio-membrane is presented in a flexible form. The membrane could be covered by the composition of the present invention to confer mechanical rigidity upon solidification.

Another possible application of the composition of the present invention is the connection of rigid and soft materials including natural or synthetic bone and metals such as titanium and its alloys in a surgical procedure. The composition can strongly hold together two pieces of bone fragments, two metal surfaces or also a piece of a bone and a metal surface.

Another possible application of the composition of the present invention is the stabilization of bone fractures: the composition of the present invention can enhance or replace a surgical or nonsurgical treatment to fix and repair bone fractures.

Another possible application of the composition of the present invention is a delivery of a drug selected from the group comprising a growth factor drug, an antiinflammatory drug, an antibiotic, other antimicrobial substances and substances supporting the healing process or their combination. The composition of the present invention contains the above drugs and acts as a delivery system. As such, the fluidity of the composition allows complete filling of a cavity to be treated and in situ hardening leads to sustained release of the drug to maintain its effect over a prolongated period. The drugs can also be pre-encapsulated in the composition of the present invention or in other types of materials. This results in a structured delivery system, allowing a controlled release delivery of at least one drug.

The composition of the present invention is produced by the following steps:
a. Providing the following powder components : glucuronic acid-1-phosphate, TTCP and a cross-linked collagen.
b. Providing water.
c. Bringing the powder components of step a. and water into contact and mixing them to provide a composition.
d. Extruding the composition of step c.

In the preferred embodiment of the present invention, the cross-linked collagen is a sugar cross-linked collagen.

### EXAMPLES

### Example 1: Composition preparation

First, the single powder components were weighed i.e. TTCP (293 mg, 0.80 mmol), glucuronic acid-1-phosphate (185 mg, 0.68 mmol) and a sugar cross-linked collagen (5 mg) and then were introduced in a capsule. Before weighing, the sugar cross-linked collagen was grinded and sieved to the particle size of 80 µm. The d(50) of TTCP was 6.5 µm. Then, into a separate compartment of the capsule, water (87 µl) was provided. The powder components and water were brought into contact by activating the capsule and mixed for 30 seconds to obtain the composition with the appearance of white putty. Finally, the composition was extruded using a capsule extruder.

## Claims

1. A composition comprising:
a. Glucuronic acid-1-phosphate
b. Tetracalcium phosphate
c. A cross-linked collagen
d. Water

2. The composition of claim 1, wherein the composition comprises 40-70 weight % of tetracalcium phosphate, 15-40 weight % of glucuronic acid-1-phosphate, 10-25 weight % of water and 0.1-5.0 weight % of cross-linked collagen, based on the total weight of the composition.

3. The composition of claims 1- 2, wherein the weight ratio of glucuronic acid-1-phosphate and tetracalcium phosphate is 0.63.

4. The composition of claims 1 -3, wherein the cross-linked collagen is a sugar cross-linked collagen.

5. The composition of claims 1 -4, wherein the weight ratio liquid /powder of the composition is 0.18.

6. The composition of claims 1 -5, wherein the Ca/P ratio of the mineral part of the composition is 2.

7. The composition of claims 1-6, wherein the d(50) of tetracalcium phosphate ≤ 10µm, measured by laser granulometry.

8. A process for producing the composition according to claims 1-7, comprising the following steps:
a. Providing the following powder components: glucuronic acid-1-phosphate, tetracalcium phosphate and a cross-linked collagen.
b. Providing water.
c. Bringing the powder components of step a. and water into contact and mixing them to provide a composition.
d. Extruding the composition of step c.

9. A bone graft material comprising the composition of claims 1-7.

10. The composition of claims 1-7 in combination with another bone graft material for use in the method of producing a bone graft.

11. The composition of claims 1-7 for use in the method of the stabilization of a dental implant.

12. The composition of claims 1-7 for use in the method of the dental ridge preservation.

13. The composition of claims 1-7 for use in the method of treatment of vertical and horizontal bone loss.

14. The composition of claims 1-7 for use in the method of treatment of larger bone defects.

15. The composition of claims 1-7 for use in the method of structural stabilization of a soft bio-membrane comprising a synthetic or a natural polymer.

16. The composition of claims 1-7 for use in the method of connection of rigid and soft materials including a natural or a synthetic bone and metals such as titanium and its alloys in a surgical procedure.

17. The composition of claims 1-7 for use in the method of stabilization of bone fractures.

18. The composition of claims 1-7 for use in the method of controlled release delivery of at least one drug.

19. Glucuronic acid-I-phosphate for use in the method of treatment in the dental and medical practice.

## Patentansprüche

1. Zusammensetzung, umfassend:
a. Glucuronsäure-1-phosphat,
b. Tetracalciumphosphat,
c. ein vernetztes Kollagen,
d. Wasser.

2. Zusammensetzung nach Anspruch 1, wobei die Zusammensetzung 40-70 Gew.-% Tetracalciumphosphat, 15-40 Gew.-% Glucuronsäure-1-phosphat, 10-25 Gew.-% Wasser und 0,1-5,0 Gew.-% vernetztes Kollagen auf Basis des Gesamtgewichts der Zusammensetzung umfasst.

3. Zusammensetzung nach Anspruch 1-2, wobei das Gewichtsverhältnis von Glucuronsäure-1-phosphat und Tetracalciumphosphat 0,63 ist.

4. Zusammensetzung nach Anspruch 1-3, wobei das vernetzte Kollagen ein zuckervernetztes Kollagen ist.

5. Zusammensetzung nach Anspruch 1-4, wobei das Gewichtsverhältnis Flüssigkeit/Pulver der Zusammensetzung 0,18 ist.

6. Zusammensetzung nach Anspruch 1-5, wobei das Ca/P-Verhältnis des mineralischen Teils der Zusammensetzung 2 ist.

7. Zusammensetzung nach Anspruch 1-6, wobei der d(50) von Tetracalciumphosphat ≤ 10 µm ist, gemessen durch Lasergranulometrie.

8. Prozess zum Produzieren der Zusammensetzung nach Anspruch 1-7, umfassend die folgenden Schritte:
a. Bereitstellen der folgenden Pulverkomponenten: Glucuronsäure-1-phosphat, Tetracalciumphosphat und ein vernetztes Kollagen,
b. Bereitstellen von Wasser,
c. Inkontaktbringen der Pulverkomponenten aus Schritt a. und Wasser und Mischen derselben, um eine Zusammensetzung bereitzustellen,
d. Extrudieren der Zusammensetzung aus Schritt c.

9. Knochentransplantatmaterial, umfassend die Zusammensetzung nach Anspruch 1-7.

10. Zusammensetzung nach Anspruch 1-7 in Kombination mit einem anderen Knochentransplantatmaterial zur Verwendung in dem Verfahren zum Produzieren eines Knochentransplantats.

11. Zusammensetzung nach Anspruch 1-7 zur Verwendung in dem Verfahren der Stabilisierung eines Zahnimplantats.

12. Zusammensetzung nach Anspruch 1-7 zur Verwendung in dem Verfahren der Zahnleistenkonservierung.

13. Zusammensetzung nach Anspruch 1-7 zur Verwendung in dem Verfahren zur Behandlung von vertikalem und horizontalem Knochenverlust.

14. Zusammensetzung nach Anspruch 1-7 zur Verwendung in dem Verfahren zur Behandlung von größeren Knochendefekten.

15. Zusammensetzung nach Anspruch 1-7 zur Verwendung in dem Verfahren zur strukturellen Stabilisierung einer weichen Biomembran, die ein synthetisches oder ein natürliches Polymer umfasst.

16. Zusammensetzung nach Anspruch 1-7 zur Verwendung in dem Verfahren zur Verbindung von starren und weichen Materialien, beinhaltend einen natürlichen oder einen synthetischen Knochen und Metalle wie Titan und seine Legierungen in einem chirurgischen Vorgang.

17. Zusammensetzung nach Anspruch 1-7 zur Verwendung in dem Verfahren zur Stabilisierung von Knochenbrüchen.

18. Zusammensetzung nach Anspruch 1-7 zur Verwendung in dem Verfahren zur Abgabe von zumindest einem Arzneimittel mit kontrollierter Freisetzung.

19. Glucuronsäure-1-phosphat zur Verwendung in dem Verfahren zur Behandlung in der zahnärztlichen und medizinischen Praxis.

## Revendications

1. Composition comprenant :
a. de l'acide glucuronique-1-phosphate
b. du phosphate de tétracalcium
c. du collagène réticulé
d. de l'eau.

2. Composition de la revendication 1, ladite composition comprenant 40 à 70 % en poids de phosphate de tétracalcium, 15 à 40 % en poids d'acide glucuronique-1-phosphate, 10 à 25 % en poids d'eau et 0,1 à 5,0 % en poids de collagène réticulé, sur la base du poids total de la composition.

3. Composition des revendications 1 à 2, dans laquelle le rapport pondéral d'acide glucuronique-1-phosphate et de phosphate de tétracalcium est de 0,63.

4. Composition des revendications 1 à 3, dans laquelle le collagène réticulé est un collagène réticulé par sucre.

5. Composition des revendications 1 à 4, dans laquelle le rapport pondéral liquide/poudre de la composition est de 0,18.

6. Composition des revendications 1 à 5, dans laquelle le rapport Ca/P de la partie minérale de la composition est de 2.

7. Composition des revendications 1 à 6, dans laquelle le d(50) de phosphate de tétracalcium ≤ 10 µm, mesuré par granulométrie laser.

8. Processus permettant la production de la composition selon les revendications 1 à 7, comprenant les étapes suivantes :
a. fourniture des composants en poudre suivants : acide glucuronique-1-phosphate, phosphate de tétracalcium et collagène réticulé,
b. fourniture d'eau,
c. mise en contact des composants en poudre de l'étape a. avec de l'eau et mélange de ceux-ci pour obtenir une composition,
d. extrusion de la composition de l'étape c.

9. Matériau de greffe osseuse comprenant la composition des revendications 1 à 7.

10. Composition des revendications 1 à 7 en combinaison avec un autre matériau de greffe osseuse, destinée à être utilisée dans le procédé de production d'une greffe osseuse.

11. Composition des revendications 1 à 7, destinée à être utilisée dans le procédé de stabilisation d'un implant dentaire.

12. Composition des revendications 1 à 7, destinée à être utilisée dans le procédé de préservation de crête dentaire.

13. Composition des revendications 1 à 7, destinée à être utilisée dans le procédé de traitement de la perte osseuse verticale et horizontale.

14. Composition des revendications 1 à 7, destinée à être utilisée dans le procédé de traitement de défauts osseux plus importants.

15. Composition des revendications 1 à 7, destinée à être utilisée dans le procédé de stabilisation structurelle d'une bio-membrane molle comprenant un polymère synthétique ou naturel.

16. Composition des revendications 1 à 7, destinée à être utilisée dans le procédé de liaison de matériaux rigides et mous comprenant un os naturel ou synthétique et des métaux tels que le titane et ses alliages dans une procédure chirurgicale.

17. Composition des revendications 1 à 7, destinée à être utilisée dans le procédé de stabilisation de fractures osseuses.

18. Composition des revendications 1 à 7, destinée à être utilisée dans le procédé d'administration à libération contrôlée d'au moins un médicament.

19. Acide glucuronique-1-phosphate destiné à être utilisé dans le procédé de traitement dans la pratique dentaire et médicale.
